(19) Europäisches Patentamt
European Patent Office
Office européen des brevets

(11) **EP 1 025 259 B1**

(12) **FASCICULE DE BREVET EUROPEEN**

(45) Date de publication et mention
de la délivrance du brevet:
**21.12.2005 Bulletin 2005/51**

(21) Numéro de dépôt: **98945369.1**

(22) Date de dépôt: **23.09.1998**

(51) Int Cl.⁷: $C12Q\ 1/22$, $C12Q\ 1/04$

(86) Numéro de dépôt international:
**PCT/FR1998/002045**

(87) Numéro de publication internationale:
**WO 1999/016896 (08.04.1999 Gazette 1999/14)**

(54) **PROCEDE DE REGULATION DE LA DESINFECTION DE LIQUIDES**

REGLUNGSPROZESS FÜR DIE DESINFIZIERUNG VON FLÜSSIGKEITEN

METHOD FOR ADJUSTING AND DISINFECTING LIQUIDS

(84) Etats contractants désignés:
**DE ES FR GB**

(30) Priorité: **29.09.1997 FR 9712082**

(43) Date de publication de la demande:
**09.08.2000 Bulletin 2000/32**

(73) Titulaire: **Suez Lyonnaise des Eaux**
**92753 Nanterre Cedex (FR)**

(72) Inventeurs:
• **LEVI, Yves**
**F-78250 Mézy sur Seine (FR)**
• **TOUATI, Danièle**
**F-75005 Paris (FR)**
• **DUKAN, Sam**
**F-95800 Cergy Saint Christophe (FR)**

(74) Mandataire: **Peaucelle, Chantal et al**
**Cabinet Armengaud Ainé**
**3, avenue Bugeaud**
**75116 Paris (FR)**

(56) Documents cités:
**EP-A- 0 574 977**        **WO-A- 95/08639**
**FR-A- 2 638 170**        **US-A- 5 158 973**
**US-A- 5 223 401**        **US-A- 5 223 402**
**US-A- 5 366 872**

• **DATABASE WPI Week 9847 Derwent**
**Publications Ltd., London, GB; AN 98-549791**
**XP002092786 "Method for quantitative determn.**
**of oxidative stress on microorganisms caused**
**by ozone treatment - by determn. of activity or**
**amount of superoxide dismutase in cells of**
**microorganisms, partic. for investigation of**
**bactericidal or bacteriostatic effect" & JP 10**
**243798 A (MITSUBISHI ELECTRIC CORP), 14**
**septembre 1998**
• **R. J. STRETTON, T. W. MANSON: "Some**
**Aspects of the Mode of Action of the**
**Antibacterial Compound Bronopol**
**(2-bromo-2-nitropropan-1,3-diol)" JOURNAL OF**
**APPLIED BACTERIOLOGY, vol. 36, no. 1, mars**
**1973, pages 61-76, XP002067066**

EP 1 025 259 B1

**Description**

La présente invention est, de manière générale, relative à des moyens pour la régulation de la désinfection de liquides. Elle concerne plus particulièrement des moyens de régulation mettant en oeuvre la mesure d'activités enzymatiques.

La garantie de qualité et d'innocuité de liquides destinés à être en contact avec un homme ou un animal, tels qu'une eau destinée à la consommation, un liquide alimentaire, une eau de baignade, une eau destinée à des préparations pharmaceutiques ou biotechnologiques, dépend directement de la fiabilité et de la sensibilité des techniques employées pour mesurer le nombre de microorganismes survivants dans ledit liquide.

Les méthodes actuellement employées pour la régulation de la désinfectio n d'un liquide utilisent les techniques de culture sur gélose et/ou les techniques de microscopies.

Les techniques de culture sur gélose consistent à compter le nombre de colonies bactériennes se développant sur différents milieux nutritifs gélosés normalisés (*e.g.* norme française NF T 90-414, Essais des Eaux, Recherche et dénombrement des coliformes et des coliformes thermotolérants). Ces techniques présentent plusieurs inconvénients.

On peut en premier lieu citer le fait qu'elles ne donnent leurs résultats qu'au bout de 24 heures en moyenne, ce qui diffère d'autant l'éventuel ajustement du procédé de désinfection.

Les techniques de culture sur gélose contraignent, de plus, à la réalisation de batteries de cultures microbiologiques pour l'analyse de chaque échantillon de liquide. En effet, toutes les familles microbiennes, et bactériennes en particulier, ne se développent pas sur le même milieu nutritif ; ainsi, on peut ne détecter aucun coliforme après culture sur le milieu nutritif normalisé pour la détection des coliformes, mais trouver bon nombre d'autres bactéries après culture sur d'autres milieux nutritifs. Le choix des milieux nutritifs gélosés détermine donc la qualité de l'analyse. Ce choix est d'autant plus délicat qu'il a parfois été observé un nombre plus important de colonies après culture sur un milieu autre que le milieu nutritif normalisé pour la détection de ces mêmes colonies. Il a par exemple été démontré que les dénombrements de la flore bactérienne aérobie revivifiable étaient plus importants sur milieu gélosé R2A que sur le milieu nutritif normalisé correspondant (voir par exemple "A new rapid medium for enumeration and subculture of bacteria from potable water" de Reasoner D. J. et Godreich E.F., App. Environm. Microbiol. (1985) 49-p.1-7). Pour établi r un diagnostic négatif fiable, les techniques de culture sur gélose appellent donc à une multiplication des analyses. Les techniques de culture sur gélose ne permettent de plus pas aisément d'automatiser la régulation du procédé de désinfection.

Les bactéries sont de plus capables, et en particulier à la suite d'un stress environnemental tel que l'application d'un procédé de désinfection, d'adopter une forme de résistance sous laquelle elles ne se multiplient plus tout en assurant un métabolisme minimal ; dès restauration de conditions environnementales plus favorables, ces bactéries pourraient reprendre leur multiplication. De telles bactéries sont dites « non cultivables mais viables » : elles ne sont pas détectables par les techniques de culture sur gélose classiques et peuvent représenter un risque biologique pour le consommateur.

Une méthode actuelle disponible pour contrôler de manière quasi-certaine l'efficacité de la désinfection d'un liquide utilise des techniques de microscopies associées à des colorations spécifiques. Cette méthode permet une discrimination entre bactéries cultivables, bactéries non cultivables mais viables et bactéries mortes. Elle nécessite cependant la mise en oeuvre de plusieurs tests de colorations pour chaque échantillon et est techniquement difficilement automatisable.

Plus particulièrement, les brevets US-A-5 223 401, US-A-5 366 872 et la demande FR-A-2 638 170 décrivent des indicateurs de stérilité. Or, la stérilisation représente un cas particulier de désinfection. Cependant, les indicateurs divulgués dans ces documents ne permettent d'obtenir que des résultats finaux qualitatifs.

Par ailleurs, le brevet US-A-5 158 973 est relatif à l'optimisation pour un échantillon donné, de la concentration de deux agents bactéricides en fonction du taux d'incorporation de $^{14}$C dans des cellules bactériennes.

La présente invention vise à pallier les inconvénients des techniques de l'art antérieur et propose un procédé de régulation de la désinfection d'un liquide, caractérisé en ce qu'il comprend :

A. à une étape de ladite désinfection ci-après désignée étape 2, la mesure de l'activité d'au moins une enzyme par mise en contact des microorganismes éventuellement présents dans ledit liquide avec un substrat choisi comme étant capable de révéler l'activité de cette ou ces enzyme(s), notamment par transformation dudit substrat en composés colorés, fluorescents ou luminescents ou par disparition dudit substrat, cette activité enzymatique étant ci-après nommée activité propre,

B. à une étape ci-après désignée étape 1, antérieure à ladite étape 2, la mesure de l'activité de la ou des même(s) enzyme(s) qu'en A, cette activité étant ci-après désignée activité initiale,

C. la traduction, pour chaque enzyme, des di tes activité propre et activité initiale, en taux de microorganismes survivants dans le ledit liquide à l'étape 2 de ladite désinfection, et cela par l'intermédiaire d'un système de référence, pré-établi à l'aide d'un échantillon dudit liquide prélevé à ladite étape 1 puis exposé à des doses de désinfectant(s) croissantes, ainsi que

D. l'ajustement, en fonction dudit taux de microorganismes survivants, de la nature et/ou des doses du (des) agent(s) chimique(s) ou physique(s) utilisé (s) pour ladite désinfection.

Par microorganismes survivants, nous entendons dans la présente demande microorganismes cultivables et/ou microorganismes non cultivables mais viables.

Par taux de microorganismes survivants, nous entendons dans la présente demande le rapport entre concentration en microorganismes survivants dans ledit liquide à ladite étape 2 de désinfection et la concentration en microorganismes survivants dans le même liquide à ladite étape 1. Ce taux de microorganismes survivants est préférentiellement exprimé en valeurs d' abattement sous la forme de puissances négatives de 10, ou en log d'abattement qui correspond à - $\log_{10}$ (abattement).

Ladite étape 1 peut, par exemple, correspondre à une étape "avant désinfection" dudit liquide et ladite étape 2 à une étape quelconque du procédé de désinfection, (étapes "après désinfection" dudit liquide y compris).

Le procédé selon l'invention s' adresse à des liquides dans lesquels les microorganismes éventuellement présents sont soumis à des conditions tout-à-fait particulières, à savoir des conditions de désinfection, induisant un stress notable des cellules.

Le procédé de la régulation de la désinfection d'un liquide selon l'invention s'adresse particulièrement aux liquides destinés à être mis en contact avec un homme ou un animal, que cela soit par simple contact, absorption, ingestion, instillation ou injection. Il s' applique particulièrement à des liquides destinés à être en contact avec un homme ou un animal tel qu' une eau de baignade, une eau destinée à la consommation, une eau destinée à des préparations pharmaceutiques ou biotechnclogiques, ou un liquide alimentaire.

Ladite mise en contact des microorganismes éventuellement présents dans ledit liquide avec ledit substrat peut être réalisée par mise en contact directe dudit liquide ou d'un échantillon dudit liquide avec ledit substrat, ou bien par mise en contact d' un concentré desdits microorganismes éventuellement présents tel que filtrat, ou culot de centrifugation, dudit liquide ou échantillon de liquide, avec ledit substrat.

Préalablement à la mesure de l'activité de certaines enzymes telles que la glucose-6-phosphate déshydrogénase ou la glutathion réductase, le procédé selon l'invention comprend, de manière avantageuse, l'étape de faire subir audit liquide, échantillon de liquide, ou concentré, un traitement de lyse, notamment par sonication.

Préalablement à la mesure de l'activité d'autres enzymes telles que catalase ou superoxyde dismutase, l'étape de lyse préalable peut être évitée : l'activité de ces enzymes peut être mesurée à l'aide d'un substrat diffusant à l'intérieur des microorganismes, tel que la lucigénine et le peroxyde d'hydrogène.

Selon un aspect avantageux de l'invention, la ou les enzyme (s) dont la (ou les) activité(s) est (sont) mesurée(s) présente (ent), dans ledit liquide, échantillon de liquide, ou concentré, un rapport entre activité propre et activité initiale en relation affine, et avec une pente significative différente de zéro, préférentiellement inférieure à -0,2, avec le taux de microorganismes survivants sur au moins une zone de valeurs desdits taux de microorganismes survivants. C'est par exemple le cas de la glucose-6-phosphate déshydrogénase pour des log d'abattement compris entre 0 et 3 environ, de la glutathion réductase pour des log d'abattement compris entre 4 et 7 environ, de la superoxyde dismutase pour des log d'abattement compris entre 3 et 6 environ.

D'autres enzymes d'intérêt peuvent notamment faire partie de la famille des déshydrogénases ou de la famille des enzymes impliquées dans la réponse au stress oxydant.

Pour déterminer si une (ou des) enzyme(s) est (sont) appropriée(s) à la mise en oeuvre de la méthode de régulation selon l'invention sur un liquide donné, il pourra être procédé comme suit :

- prélèvement d'au moins un échantillon dudit liquide et mesure de la concentration en microorganismes survivants (microorganismes cultivables et/ou microorganismes non cultivables mais viables), notamment par culture surgélose et/ou par colorations et observations au microscope,

- dosage de l'activité de chaque enzyme candidate, sur cet cu ces échantillon (s) de liquide

- exposition dudit (desdits) échantillon(s) de liquide à différentes doses de désinfectant(s) dans des conditions par ailleurs équivalentes (*e.g.* conditions de durée d' exposition, de température),

- mesure de l'activité de chaque enzyme candidate après exposition aux différentes doses de désinfectant(s),

- réalisation, pour chaque enzyme candidate, d'une courbe présentant les pourcentages d'activité (activité enzymatique mesurée, après exposition aux différentes doses de désinfectant (s) rapportée à l'activité enzymatique correspondante avant exposition) en fonction des log d'abattement mesurés (tels que ci-avant définis),

- détermination, pour chaque enzyme candidate, de la zone de log d'abattement pour laquelle la pente de ladite courbe est significativement différente de zéro, préférentiellement inférieure à -0,2, cette zone constituant la gamme de réponse de l'enzyme candidate considérée sur ledit liquide,

- une enzyme candidate est appropriée à la mise en oeuvre de la méthode selon l'invention sur ledit liquide si sa gamme de réponse comprend les valeurs de log d'abattement correspondant aux objectifs de désinfection visés pour le liquide considéré.

Selon un aspect particulièrement avantageux de l'invention, la ou au moins une des enzyme(s) dont la (ou les) activité(s) est (sont) mesurée(s) est une glucose-6-phosphate deshydrogénase; une malate déshydrogénase, une glycéraldéhyde-3-phosphate déshydrogénase, une catalase, une superoxyde dismutase ou une glutathion reductase. Ladi te (ou au moins une desdites) activité(s) enzymatique(s) est (sont) alors mesurée(s) par transformation d' un substrat et le cas échéant, en présence d'un co-facteur, tel que respectivement glucose-6-phosphate et co-facteur NADP, L-malate et co-facteur NAD, glycéraldéhyde-3-phosphate et co-facteur NAD, lucigénine, peroxyde d'hydrogène, glutathion oxydé et co-facteur NADPH.

Selon un mode avantageux de réalisation de l'invention, lesdites mesures d'activité propre et d'activité initiale sont faites à l'aide d'un appareil de détection d'intensite lumineuse tel que spectrophotomètre, spectrofluoromètre, ou luminometre, présentant éventuellement plusieurs canaux d'analyse.

Selon un autre mode avantageux de réalisation, de l'invention, ladite traduction en taux de microorganismes survivants à l'aide dudit système de référence pré-établi comprend, pour chaque enzyme, le calcul du rapport entre activité propre et activité initiale, éventuellement exprimé en pourcentage.

Selon encore un autre mode avantageux de réalisation de l'invention, ledit système de référence se présente sous une forme graphique telle qu' une ou plusieurs courbe(s) mettant, pour chaque enzyme, ledit rapport entre activité propre et activité initiale en relation avec des valeurs de taux de microorganismes survivants tels que des log d'abattement. Ledit rapport entre activité propre et activité initiale est également désignée par "activité relative" dans la présente demande.

Selon un aspect de cet autre mode avantageux de réalisation de l'invention, lesdites mesures spectrométriques peuvent notamment être réalisées, pour chaque activité enzymatique mesurée, à température constante, notamment à 25°C, et à longueur d'onde constante, notamment à une longueur d'onde comprise entre 240 et 550 nm, par exemple à 340 nm. Lesdites mesures peuvent être enregistrées de manière continue ou discrète sur un intervalle de temps de 30 min environ. Dans le cas où l'on recherche des sensibilités de mesure plus élevées, la luminométrie est préférentiellement utilisée.

L'étape d'ajustement de désinfection du procédé selon l'invention peut se faire par suivi régulier de l'évolution du taux de microorganismes survivants (*e.g.* exprimé en abattement, log d'abattement ou indice D) à l'aide des indicateurs enzymatiques ci-avant présentés, et cela jusqu'à obtention de l'objectif de désinfection fixé.

L'étape d'ajustement de désinfection du procédé selon l'invention peut également se faire par ajout de l'équivalent "dose de désinfectant (s)" correspondant à la différence entre le taux de microorganismes survivants visé (valeur consigne) et le taux de microorganismes effectivement mesuré sur ledit liquide, échantillon de liquide, ou concentré, à une étape de ou après désinfection.

Avantageusement, ledit équivalent dose de désinfectant(s) est tel que lu sur une courbe de référence représentant le taux de microorganismes cultivables en fonction de la dose de désinfectant(s) à laquelle ledit liquide est exposé.

L'invention donne des résultats particulièrement avantageux pour des liquides comprenant des microorganismes choisis parmi le groupe constitué notamment par le genre *Escherichia, Alcaligenes, Bacillus, Flavobacterium, Methylobacterium, Pseudomonas, Klebsiella, Enterobacterium, Agrobacterium, Streptococcus, Micrococcus, Salmonella.*

Le procédé de régulation de la désinfection d'un liquide selon l'invention peut, de manière particulièrement avantageuse, être facilement automatisé sur un procédé de désinfection de liquide. Contrairement aux techniques de l'art antérieur, le procédé selon l'invention permet un contrôle microbiologique sûr, rapide et aisé à mettre en oeuvre.

De manière préférentielle, ledit taux de microorganismes survivants est exprimé en valeur d'abattement (concentration en microorganismes survivants rapportée à la concentration initiale en microorganismes survivants, telle que mesurée à ladite étape 1, de log d'abattement ($-\log_{10}$ (abattement)) ou d'indice D (= log d'abattement). Ledit indice D constitue également un indice de la qualité microbiologique du liquide considéré.

Ledit procédé de régulation de la désinfection d'un liquide selon l'invention considère, comme microorganismes survivants, ceux des microorganismes présents qui sont cultivables et/ou ceux des microorganismes présents qui ne sont pas cultivables mais qui sont viables. Dans le cas où l'on souhaite tenir compte à la fois des microorganismes cultivables et des microorganismes non cultivables mais viables, ledit système de référence met alors avantageusement, pour chaque enzyme, ledit rapport entre activité propre et activité initiale en relation avec des valeurs de taux de microorganismes survivants résultant de l'addition des valeurs de taux de microorganismes cultivables et des valeurs de taux de micrcorganismes non cultivables mais viables, tels que mesurés à l'aide des techniques classiques.

Parmi lesdits agents chimiques ou physiques avantageusement utilisés pour ladite désinfection, peuvent être cités le chlore et ses dérivés, les UV, l'ozone, $H_2O_2$, les membranes filtrantes, la température, les ultra-sons, les rayonnements ionisants.

D'autres caractéristiques et avantages de la présente invention apparaîtrons encore à travers les exemples de réalisation suivants, donnés à titre indicatif et non limitatifs.

Lesdits exemples font référence aux figures 1, 2 et 3 :

La figure 1 représente le taux de bactéries *Escherichia coli* cultivables (exprimé en valeur d'abattement par rapport à la population initiale) en fonction de la concentration en chlore libre appliquée (mg/l),

La figure 2 représente l'activité glucose-6-phosphate déshydrogénase (ZWF), exprimée en % de l'activité maximale mesurée, en fonction du taux de bactéries cultivables (exprimé en valeurs d'abattement par rapport à la population microbienne initiale),

La figure 3 représente l'activité glutathion réductase, exprimée en % de l'activité initiale (maximale) mesurée, en fonction du taux de bactéries cultivables (exprimé en valeurs d'abattement par rapport à la population microbienne maximale).

### EXEMPLE 1 : Contrôle microbiologique d'une eau en désinfection destinée à la consommation humaine.

On cherche à déterminer si des activités enzymatiques peuvent constituer un indicateur fiable du taux de microorganismes survivants dans un liquide en désinfection tel qu'une eau destinée à la consommation humaine.

### Méthodes et Résultats

A ce titre, on réalise un inoculum par culture pure *d'Escherichia coli* (souche MG1655 disponible auprès de l'Institut Pasteur) sur milieu nutritif à 25°C (milieu LB comprenant 10 g/l de tryptone Bacto, 5 g/l d'extrait de levure Eacto, 10 g/l de NaCl, pH ajusté a 7). Les bactéries sont récoltées en phase exponentielle de croissance par centrifugation puis lavées à l'aide d'un tampon phosphate (pH 7 ; 0,05 M). Les culots sont remis en suspension (environ $5.10^7$ cellules/ml) dans des solutions de tampon phosphate (pH 7 ; 0, 05 M) comprenant du chlore libre à différentes concentrations (de 0,0 à 2,0 mg/l). Les différentes suspensions bactériennes sont ensuite placées sous agitation à 25 °C. Au bout de 20 min, on prélève alors pour analyses des échantillons de ces suspensions bactériennes (volume de 100 à 1 000 ml par activité enzymatique à mesurer).

Pour chaque échantillon, on mesure en parallèle le taux de microorganismes survivants et différentes activités enzymatiques selon les méthodes suivantes.

### Numération des microorganismes survivants

On compte, pour chaque échantillon de suspension bactérienne, le nombre de microorganismes qui ont survécu, *i.e.* le nombre de bactéries cultivables et/ou le nombre de bactéries non cultivables mais viables.

La numération des microorganismes survivants peut se faire selon des techniques classiques connues de l'homme du métier : par exemple, par étalement sur un milieu gélosé tel qu'un milieu de TSA (Tryptic Soy Agar, Difco) pour la numération des bactéries cultivables, et par la technique du C.T.C. pour la numération des bactéries non cultivables mais viables (Schaule G. , Flemming H. C. et Ridgway H.F. 1993, *Use of 5-cyano-2,3-ditolyl tetrazolium chloride for quantifying planktonic and sessile bacteria in drinking water*, Appl. Environ. Microbiol. 59 : 3850-3857).

A partir du nombre moyen $n_i$ mesuré, on calcule alors la concentration moyenne ($C_i$) en microorganismes survivants à chacune des doses i de chlore libre testée. Chaque concentration moyenne $C_i$ en microorganismes survivants est alors exprimée relativement à la concentration maximale en microorganismes survivants telle que mesurée avant désinfection (concentration maximale $C_{max}$). Dans le cas de la présente illustration, $C_{max}$ correspond à la concentration moyenne en microorganismes survivants telle que mesurée en l'absence de chlore libre. Chaque $C_i$ est ainsi traduite en valeur d'abattement (ou d'élimination) à l'aide de la formule :

$$abattement = C_i \, / \, C_{max}$$

Ce taux de microorganismes survivants $\dfrac{C_i}{C_{max}}$ représente également une mesure de la qualité de désinfection.

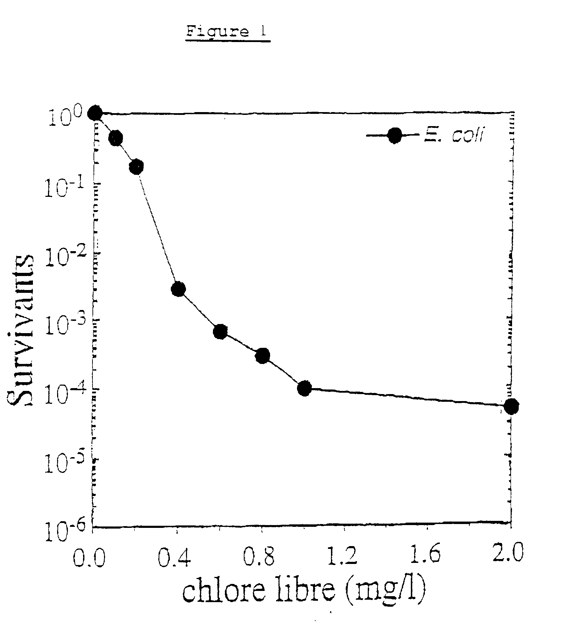

Les résultats des numérations de microorganismes cultivables sont illustrés par la figure 1 où est reporté le taux de bactéries *E. coli* cultivables en fonction de la dose en chlore libre subie. En ordonnée de la figure 1, sont reportées les valeurs d' abattement (ou d'élimination) correspondant aux concentrations en microorganismes cultivables telles qu'obtenues par numération, et rapportées à la concentration maximale mesurée avant désinfection : $10^0$ indique, une concentration en micrcorganismes cultivables égale à la concentration maximale mesurée ; $10^{-1}$ indique que la concentration en microorganismes cultivables a diminué d' un facteur de 10, $10^{-2}$ indique que la concentration en microorganismes cultivables a diminué d'un facteur de $10^{-1}$ etc. En abscisse de la figure 1, est reportée la concentration initiale en chlore libre de la suspension bactérienne correspondante. On procède à l'identique avec les résultats des numérations de microorganismes non cultivables mais viables, qui peuvent, si désiré, être additionnés aux résultats de numération des microorganismes cultivables.

### Activités enzymatiques

Parallèlement à la numération des microorganismes survivants ci-avant décrite, sont mesurées différentes activités enzymatiques.

Sont ici plus particulièrement reportées les expériences relatives à deux enzymes : la glucose-6-phosphate déshydrogénase (ci-après désignée ZWF) et la glutathion réductase. Ces enzymes sont communément présentes chez de nombreux microorganismes, elles sont donc susceptibles de pouvoir représenter l'ensemble des populations microbiennes présentes dans les suspensions et ainsi de donner l'image résultante de la désinfection réalisée.

Dans les expériences ici décrites, les bactéries en suspension sont à de trop faibles concentrations pour que leurs activités enzymatiques puissent être correctement mesurées directement sur l'échantillon liquide prélevé sans concentration préalable. Les échantillons de suspensions sont donc ici filtres (membrane de 0,22 μm) de manière à en récolter les microorganismes. Les microorganismes peuvent également être récoltés par centrifugation à 3 000 g pendant 10 min à 4°C.

Les études comparatives menées montrent qu'il est préférable de lyser les microorganismes préalablement à la mesure d'activités ZWF ou glutathion réductase. Les filtres (ou culot) sont donc ici placés dans un système permettant la lyse des microorganismes récoltés : préalablement à une mesure d'activité ZWF ou glutathion réductase, la lyse des microorganismes se fait de manière préférentielle par une sonde à ultra-sons (2 cycles de 30s sous ultra-sons et 30s en repos). On peut noter que, pour mesurer l'activité d'enzymes autres que ZWF ou glutathion réductase, telles que *e. g.* catalase ou superoxyde dismutase, la lyse préalable des microorganismes peut être évitée en utilisant un substrat diffusant tel que la lucigénine et le peroxyde d'hydrogène.

Les différentes activités enzymatiques peuvent être mes urées selon des techniques connues de l'homme du métier. Brièvement, pour chaque activité enzymatique à mesurer, les microorganismes de chaque échantillon sont placés en contact avec un substrat choisi de manière à ce que l'enzyme ciblée puisse catalyser sa transformation et à ce que cette transformation enzymatique puisse être aisément suivie par les techniques analytiques classiques telles que spectrocolorimétrie, spectrofluorométrie, ou luminométrie pour lesquelles une automatisation est réalisable.

Pour mesurer une activité glucose-6-phosphate déshydrogénase, on place les microorganismes de l'échantillon en contact avec un substrat composé de glucose-6-phosphate 0,6 mM et de nicotinamide adénine dinucléotide phosphate sous forme oxydée (NADP 0,2 mM) en présence d'une solution stabilisant le pH (ajout de tampon Tris pH 7,6 MgCl$_2$ 10mM). Ce substrat conduit, en présence de glucose-6-phosphate déshydrogénase, à la formation de nicotinamide adénine dinucléotide phosphate sous forme réduite (NADPH) dont on peut suivre l'apparition par spectrocolorimétrie à la longueur d'onde de 340 nm (Fraenkel D.G. et Levisohn S. R. 1967, *Glucose and gluconate metabolism in an Escherichia coli mutant lacking phosphoglucose isomerase*, J. Bact 93 : 1571-1578).

Pour mesure une activité glutathion réductase, on place les microorganismes de l'échantillon en contact avec un substrat composé de nicotinamide adénine dinucléctide phosphate sous forme réduite (NADPH 0,2 mM) et de glutathion oxydé (disulfure de glutathion GS-SG 2,5 mM) en présence d'une solution stabilisant le pH (tampon phosphate 100 mM pH 7). Sous l'action catalytique de la glutathion réductase, ce substrat est transformé en nicotinamide adénine dinucléotide phosphate sous forme oxydée (NADP) et en glutathion sous forme réduite (GSH). La disparition de NADPH est alors suivie au spectrocolorimètre à la longueur d'onde de 340 nm (Lopez-Barea J. et Lee C. Y. 1979, *Mouse liver glutathione reductase: purification, kinetics* and *regulation,* Eur. J. Biochem. 98 : 487-499).

Les mesures d'activités enzymatiques sont ici réalisées à une température constante identique (25°C) à l'aide d'un spectrophotomètre PERKIN-ELMER modèle lambda 1.

La valeur de densité optique mesurée avant le démarrage de la réaction enzymatique considérée sert de "blanc de mesure". Les valeurs de densités optiques propres de chaque milieu réactionnel sont alors enregistrées au cours du temps.

L'activité enzymatique propre de l'échantillon est ensuite calculée dans la partie linéaire de la courbe représentant la densité optique propre observée après la mise en contact du substrat avec l'enzyme ciblée (par exemple, entre 5 min et 35 min). Le calcul de la pente de la courbe "densité optique propre de l'échantillon en fonction du temps" dans l'intervalle de temps 5-35 min considéré donne une valeur de cette variation de densité optique propre.

Pour chaque enzyme, les activités enzymatiques propres mesurées aux différentes doses de désinfectants (quantité de substrat consommé ou produit par unité de temps) sont chacune exprimées en % de la valeur initiale d'activité enregistrée pour la même enzyme, *i.e.* en % de la valeur d'activité mesurée, pour la même enzyme, à la plus faible dose de désinfectant : dans la présente illustration, l'activité propre glucose-6-phosphate déshydrogénase (ZWF) et l'activité propre glutathion réductase des échantillons sont exprimées en % de l'activité propre glucose-6-phosphate déshydrogénase (ZWF) et, respectivement, glutathion réductase telle que mesurée pour les suspensions bactériennes ne comportant pas de chlore libre (i.e. avant désinfection). Ce rapport entre activité enzymatique propre du liquide à une étape du procédé de désinfection (i.e. en cours ou après désinfection) et activité enzymatique propre de ce même liquide avant désinfection est ici désigné activité enzymatique relative du liquide à ladite étape de désinfection.

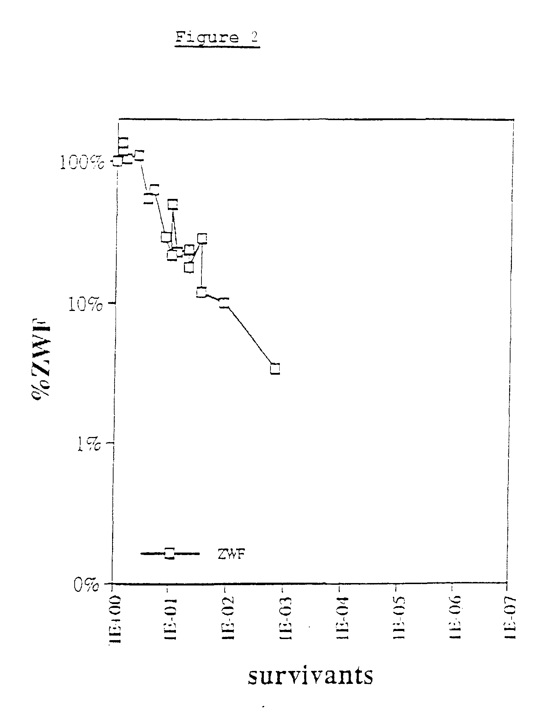

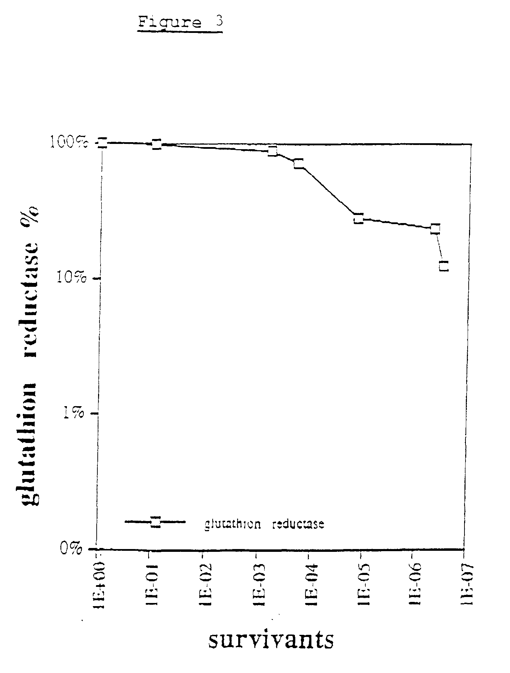

Les activités enzymatiques relatives obtenues sont alors comparées aux mesures de numérations microbiologiques. Les résultats des numérations de microorganismes cultivables sont illustrés par les figures 2 et 3.

La figure 2 représente, en ordonnée, l'activité glucose-6-phosphate déshydrogénase (ZWF) mesurée, exprimée en % du maximum (activité initiale) d'activité enregistré, et, en abscisse, le nombre correspondant de *E. coli* cultivables obtenu par numération.

La figure 3 représente, en ordonnée, l'activité glutathion réductase mesurée, exprimée en % du maximum d' activité enregistré (activité initiale), et, en abscisse, le nombre correspondant de *E. coli* cultivables obtenu par numération.

En figure 2 tout comme en figure 3, le nombre de microorganismes survivants est exprimé en fonction du log d'abattement subi selon la formule suivante :

$$\log \text{d'abattement} = - \log_{10} (C_i / C_{max})$$

où $C_i$ et $C_{max}$ sont tels que définis ci-avant.

Si nécessaire, les mêmes types de figures peuvent être réalisées en tenant compte des résultats des numérations de microorganismes non cultivables mais viables.

En figure 2 tout comme en figure 3, les valeurs de log d' abattement sont reportées sur l'axe des abscisses de la manière suivante : 1E+00 représente un nombre de microorganismes cultivables égal au nombre maximal enregistré (suspension ne présentant pas de chlore libre) ; 1E-01 représente un nombre de microorganismes cultivables égal au nombre maximal enregistré diminué d'un nombre de microorganismes correspondant à une valeur de log d'abattement de 1 ; 1E-02 représente un nombre de microorganismes cultivables égal au nombre maximal enregistré diminué d'un nombre de microorganismes correspondant à une valeur de log d'abattement de 2, et ainsi de suite jusqu'à 1E-07 qui représente un nombre de microorganismes cultivables égal au nombre maximal enregistré diminué d'un nombre de microorganismes correspondant à une valeur de log d'abattement de 7.

On peut noter que cette valeur de log d'abattement constitue également un indice de la qualité microbiologique du liquide considéré, indice que nous nommons D.

Les résultats obtenus montrent que le suivi de l'activité glucose-6-phosphate déshydrogénase (activité relative; est un indicateur représentatif du nombre de microorganismes survivants pour une gamme d'échantillons allant des échantillons de liquide n'ayant subi aucun traitement d'élimination des microorganismes jusqu'aux échantillons de liquide présentant un log d'abattement (ou d' élimination) inférieur ou égal à 3 environ. (cf. figure 2).

Les résultats obtenus montrent également que l'activité glutathion réductase (activité relative) est un indicateur représentatif du nombre de microorganismes survivants pour une gamme d'échantillons de liquide présentant un log d'abattement (ou d'élimination) compris entre 4 et 7 environ. (cf. figure 3). En effet, l'activité de la glutathion réductase n'est pas significativement affectée avant que ne soient atteints 4 log d'abattement. Une proportionnalité significative entre activité relative et log d'abattement n'est observée, pour cette enzyme, que sur la zone des log d'abattement compris entre 4 et 7 environ.

De manière similaire, nous avons pu démontrer que l'activité (relative) superoxyde dismutase (mesures en luminométrie à l'aide de lucigénine) est un indicateur représentatif du nombre de microorganismes survivants pour une gamme d'échantillons de liquide présentant un log d'abattement (ou d'élimination) compris entre 3 et 6 envi ron. Les superoxydes dismutases et les catalases présentent de plus l'avantage de ne pas nécessiter de traitement de lyse : la mesure de leur activité peut être réalisée sur un substrat diffusant tel que la lucigénine, ou respectivement le peroxyde d'hydrogène, par luminométrie.

Les différentes enzymes testées ne couvrent donc pas les mêmes domaines de sensibilité : l'activité relative de la glucose-6-phosphate déshydrogénase (ZWF) est un indicateur représentatif du nombre de microorganismes survivants (log d'abattement) dans des échantillons de liquide n'ayant subi que de faibles diminutions relatives de populations microbiennes, l'activité relative de la superoxyde dismutase et de la glutathion réductase sont des indicateurs représentatifs du nombre de microorganismes survivants (log d'abattement) dans des échantillons de liquide ayant subi des diminutions relatives de populations microbiennes moyennes à fortes.

Les mêmes types de gammes de sensibilité (proportionnalité entre activité relative et log d'abattement pour certaines zones de valeurs de log d'abattement) ont pu être observés en mesurant l'activité relative de la malate déshydrogénase, la glycéraldéhyde-3-phosphate déshydrogénase, les catalases (soit par mesure de la consommation de peroxyde d'hydrogène à 240 nm dans une solution tamponnée à pH 7 soit par chimioluminescence). L' homme du métier pourra trouver d'autres exemples d'enzymes et de protocoles de mesure d'acti vi tés enzymatiques dans différents ouvrages de référence tels que : *Oxidative stress and the molecular biology of antioxidant defenses*, 1997, Cold Spring Harbor Laboratory Press O-87969-502-1/97 ; Lehninger 1977, Biochimie, Flammarion ISBN 2-257-25009-5 ; *Methods in Enzymology*, Academic Press Inc. , *e.g.* volumes I-XLI-XLII-89-105 et 234.

Toute enzyme pour laquelle une proportionnalité significative (pente significative, par exemple inférieure à -0,2) entre activité relative et log d'abattement peut être mise en évidence, par exemple en suivant le protocole ci-dessus décrit, constitue un indicateur fiable selon l'invention.

Les mêmes types de gammes de sensibilité enzymatique peuvent être obtenus en appliquant aux suspensions de *E. coli* non pas des doses croissantes de chlore mais des doses croissantes en ozone ou des doses croissantes en UV.

## Discussion

Il apparaît donc que la mesure d'activités enzymatiques permet de suivre l'évolution des populations microbiennes dans un liquide en désinfection. Ces différents indicateurs enzymatiques de survie microbienne permettent de rendre compte de la totalité des populations microbiennes : microorganismes cultivables et microorganismes non cultivables mais viables.

On peut par exemple suivre l'activité relative ZWF pour des log d'abattement inférieurs à 3, et l'activité relative glutathion réductase pour des log d'abattement supérieurs à 4. Dans le cas où une mesure précise d'un log d'abattement compris entre 3 et 4 est requise, on peut alors par exemple mesurer une activité relative superoxyde dismutase.

Dans le cas où la désinfection effectuée n'a pas à conduire à des log d'abattement supérieurs à 6, on peut alors soit simplement suivre l'activité relative superoxyde dismutase, qui ne commencera à répondre qu'à partir de log d' abattement supérieurs à 3, soit suivre l'activité relative ZWF jusqu'aux log d'abattement de 3, puis l'activité relative superoxyde dismutase au-delà.

Les différents types d'indicateurs enzymatiques de survie microbienne ci-avant présentés permettent donc de connaître la valeur de log d'abattement du liquide contrôlé, et par là même, son indice D de qualité microbiologique, sa valeur d'abattement, et le nombre de microorganismes qui y survivent. Ils donnent donc *in fine* une mesure de la vitesse et de l'efficacité du procédé de désinfection appliqué.

Si le nombre de microorganismes survivants dans le liquide contrôlé (*e.g.* exprimé sous la forme d'un log d'abattement ou indice de qualité de désinfection) ne correspond pas à l'objectif de désinfection fixé (*e.g.* valeur consigne du log d'abattement ou de qualité de désinfection), la dose de désinfectant(s) appliquée audit liquide (*e.g.* la concentration en chlore libre, en ozone, la dose d'U.V., de température, d'ultra-sons, de rayonnements ionisants) peut être ajustée en conséquence.

Cette augmentation ou diminution de la dose de désinfectant(s) peut se faire en suivant de manière régulière l'évolution du nombre de microorganismes survivants (log d'abattement) à l'aide des indicateurs enzymatiques ci-avant présentés et cela jusqu'à obtention de l'objectif de désinfection fixé.

L'ajustement de la dose de désinfectant(s) peut également se faire à l'aide de courbes de référence préétablies sur un échantillon dudit liquide représentant le taux de microrganismes survivants, *e.g.* exprimé en valeurs de log d'abattement, en fonction de la dose de désinfectant(s) appliquée (*e.g.* doses croissantes de chlore libre, d'ozone, d' U.V., de température ultra-sons, rayonnements ionisants).

De telles courbes de référence permettent de lire les valeurs de doses de désinfectant(s) équivalentes respectivement au taux mesuré et taux désiré de microorganismes survivants tels qu'obtenus à l'aide des indicateurs enzymatiques ci-avant présentés. Il suffit alors d'augmenter ou de diminuer r la dos e de désinfectant(s) appliquée au liquide contrôlé de la différence lue entre ces équivalents doses de désinfectant(s).

De telles courbes de référence peuvent par exemple être obtenues en dénombrant les microorganismes survivants dans un échantillon dudit liquide exposé à des doses croissantes de désinfection (*e.g.* concentrations croissantes en chlore libre, en ozone, doses croissantes en U. V. de rayonnements ionisants, d'ultrasons, valeurs croissantes de température).

Le procédé de régulation de la désinfection d'un liquide selon l'invention permet donc, à l'aide de mesures d'activités enzymatiques, de contrôler de manière complète, simple et rapide (moins d'une heure) la désinfection d'un liquide, et cela quel que soit l'état physiologique ou l'identité des microorganismes qui y survivent. Le procédé de régulation de la désinfection d'un liquide selon l'invention présente de plus l'avantage particulier d'être aisément automatisable, contrairement aux procédés mettant en oeuvre les techniques de microscopies ou de cultures microbiologiques.

Il demeure bien entendu que la présente invention n'est pas limitée aux exemples de réalisation décrits et représentés ci-dessus, mais qu'elle en englobe toutes les variantes. C'est ainsi que notamment les mesures d'activités enzymatiques peuvent être réalisées par des techniques analytiques autres que celles ci-dessus mentionnées.

## Revendications

**1.** Procédé de régulation de la désinfection d' un liquide, **caractérisé en ce qu'**il comprend :

    A. à une étape de ladite désinfection ci-après désignée étape 2, la mesure de l'activité d'au moins une enzyme par mise en contact des microorganismes éventuellement présents dans ledit liquide avec un substrat choisi comme étant capable de révéler l'activité de cette ou ces enzyme(s), cette activité enzymatique étant ci-après nommée activité propre,
    B. à une étape ci-après désignée étape 1, antérieure à ladite étape 2, la mesure de l'activité de la ou des même(s) enzyme(s) qu'en A, cette activité étant ci-après nommée activité initiale,
    C. la traduction, pour chaque enzyme, desdites activité propre et activité initiale, en taux de microorganismes survivants dans le ledit liquide à l'étape 2 de ladite désinfection, et cela par l'intermédiaire d'un système de référence, préétabli à l'aide d'un échantillon dudit liquide prélevé à ladite étape 1 puis exposé à des doses de désinfectant(s) croissantes, ainsi que

D. l'ajustement, en fonction dudit taux de microorganismes survivants, de la nature et/ou des doses du (des) agent(s) chimique(s) ou physique(s) utilisé(s) pour ladite désinfection.

2. Procédé selon la revendication 1, **caractérisé en ce que** ladite étape 1 correspond à une étape avant désinfection dudit liquide et **en ce que** ladite étape 2 correspond à une étape quelconque de ladite désinfection.

3. Procédé selon la revendication 1 ou 2, **caractérisé en ce que** ledit liquide est un liquide destiné à être en contact avec un homme ou un animal tel qu'une eau de baignade, une eau destinée à la consommation, une eau destinée à des préparations pharmaceutiques ou biotechnologiques, ou un liquide alimentaire.

4. Procédé selon l'une quelconque des revendications 1 à 3, **caractérisé en ce que** ladite mise en contact des microorganismes éventuellement présents dans ledit liquide avec ledit substrat est réalisée par mise en contact dudit liquide ou d'un échantillon dudit liquide avec ledit substrat.

5. Procédé selon l'une quelconque des revendications 1 à 4, **caractérisé en ce que** ladite mise en contact des microorganismes éventuellement présents dans ledit liquide, ou échantillon de liquide, avec ledit substrat est réalisée par mise en contact d'un filtrat, ou d'un culot de centrifugation, dudit liquide ou échantillon de liquide, avec ledit substrat.

6. Procédé selon l'une quelconque des revendications 1 à 5, **caractérisé en ce que** ledit liquide, échantillon de liquide, ou concentré, subissent, préalablement auxdites mesures de l'activité d' au moins une enzyme, un traitement de lyse, notamment par sonication.

7. Procédé selon l'une quelconque des revendications 1 à 6, **caractérisé en ce que** la ou les enzyme (s) dont la (ou les) activité(s) est (sont) mesurée(s) présente (ent) , dans ledit liquide, échantillon de liquide, ou concentré, un rapport entre activité propre et activité initiale en relation affine, et avec une pente significativement différente de zéro, préférentiellement inférieure à -0,2, avec le taux de microorganismes survivants sur au moins une zone de valeurs desdits taux de microorganismes survivants.

8. Procédé selon l'une quelconque des revendications 1 à 7, **caractérisé en ce que** la ou au moins une des enzyme(s) dont la (ou les) activité(s) est (sont) mesurée (s) est une glucose-6-phosphate deshydrogénase, une malate déshyorogénase, une glycéraldéhyde-3-phosphate deshydrogénase, une catalase, une superoxyde dismutase, ou une glutathion reductase.

9. Procédé selon l'une quelconque des revendications 1 à 8, **caractérisé en ce que** lesdites lesdites mesures d'activité propre et d'activité initiale sont faites à l'aide d'un spectrophotomètre, d'un spectrofluoromètre, ou d'un luminomètre présentant éventuellement plusieurs canaux d'analyse.

10. Procédé selon l'une quelconque des revendications 1 à 9, **caractérisé en ce que** ladite traduction en taux de microorganismes survivants à l'aide dudit système de référence comprend, pour chaque enzyme, le calcul du rapport entre activité propre et activité initiale, éventuellement exprimé en pourcentage.

11. Procédé selon l'une quelconque des revendications 1 à 10, **caractérisé en ce que** ledit système de référence se présente sous une forme graphique telle qu'une ou plusieurs courbe(s) mettant, pour chaque enzyme, ledit rapport entre activité propre et activité initiale en relation avec des valeurs de taux de microorganismes survivants.

12. Procédé selon l'une quelconque des revendications 9 à 11, **caractérisé en ce que** lesdites mesures spectrométriques sont réalisées pour chaque activité enzymatique mesurée, à température constante, notamment à 25 ° C, et à longueur d'onde constante notamment à une longueur d'onde comprise entre 240 et 550 nm, par exemple à 340 nm.

13. Procédé selon l'une quelconque des revendications 9 à 12, **caractérisé en ce que** lesdites mesures spectrométriques sont réalisées de manière continue ou discrète sur un intervalle de temps de 30 min environ.

14. Procédé selon l'une quelconque des revendications 1 à 13, **caractérisé en ce que** ledit ajustement des doses du (des) agent(s) chimique(s) ou physique(s) se fait par ajout de l'équivalent "dose de désinfectant(s)" correspondant à la différence entre le taux de microorganismes survivants visé et le taux de microorganismes mesuré sur ledit liquide, échantillon de liquide, ou concentré.

15. Procédé selon la revendication 14, **caractérisé en ce que** ledit équivalent dose de désinfectant(s) est tel que lu sur une courbe de référence représentant le taux de microorganismes survivants en fonction de la dose de désinfectant(s) à laquelle ledit liquide est exposé.

16. Procédé selon l'une quelconque des revendications 1 à 15, **caractérisé en ce que** ledit liquide comprend des microorganismes choisis parmi le groupe constitué par le genre *Escherichia, Alcaligenes, Bacillus, Flavobacterium, Methylobacterium, Pseudomonas, Klebsiella, Enterobacterium, Agrobacterium, Streptococcus, Micrococcus, Salmonella.*

17. Procédé selon l'une quelconque des revendications 1 à 16, **caractérisé en ce qu'**il est automatisé sur un procédé de désinfection de liquide.

18. Procédé selon l'une quelconque des revendications 1 à 17, **caractérisé en ce que** ledit taux de microorganismes survivants est exprimé en valeur d'abattement (concentration en microorganismes survivants rapportée à la concentration initiale en microorganismes survivants, telle que mesurée à ladite étape 1, de log d'abattement (-$\log_{10}$ (abattement)) ou d'indice D (= log d'abattement).

19. Procédé selon l'une quelconque des revendications 1 à 18, **caractérisé en ce que** lesdits microorganismes survivants sont des microorganismes cultivables et/ou des microorganismes non cultivables mais viables.

20. Procédé selon l'une quelconque des revendications 1 à 19, **caractérisé en ce que** lesdits agents chimiques ou physiques utilisés pour la désinfection sont choisis parmi le groupe constitué par le chlore et ses dérivés, les UV, l'ozone, $H_2O_2$, les membranes filtrantes, la température, les ultra-sons, les rayonnements ionisants.

**Patentansprüche**

1. Verfahren zur Regelung der Desinfektion einer Flüssigkeit, **dadurch gekennzeichnet, dass** es

    a) in einer nachfolgend als Stufe 2 bezeichneten Stufe der Desinfektion die Messung der Aktivität mindestens eines Enzyms, indem gegebenenfalls in der Flüssigkeit vorhandene Mikroorganismen mit einem Substrat in Berührung gebracht werden, das in der Lage ist, die Aktivität des/der Enzyme/s nachzuweisen, wobei diese Enzymaktivität anschließend als wahre Aktivität bezeichnet wird,

    b) in einer nachfolgend als Stufe 1 bezeichneten Stufe, die der Stufe 2 vorangeht, die Messung der Aktivität desselben/derselben Enzyme/s wie in a), wobei diese Aktivität anschließend als Anfangsaktivität bezeichnet wird,

    c) das Ausdrücken von wahrer und Anfangsaktivität für das jeweilige Enzym als Anteil an Mikroorganismen, die in der Flüssigkeit in Stufe 2 der Desinfektion überlebt haben, über ein Referenzsystem, das vorher mittels einer Probe dieser Flüssigkeit hergestellt worden ist, die in Stufe 1 entnommen und anschließend steigenden Dosen an Desinfektionsmittel/n ausgesetzt wurde, und

    d) die Anpassung des Charakters und/oder der Dosen des/der chemischen bzw. physikalischen Mittel/s, das/die zur Desinfektion angewendet wird/werden, abhängig vom Anteil an überlebenden Mikroorganismen,

umfasst.

2. Verfahren nach Anspruch 1, **dadurch gekennzeichnet, dass** die Stufe 1 einer Stufe vor der Desinfektion der Flüssigkeit entspricht, **und dass** die Stufe 2 einer beliebigen Stufe der Desinfektion entspricht.

3. Verfahren nach Anspruch 1 oder 2, **dadurch gekennzeichnet, dass** die Flüssigkeit eine solche, die vorgesehen ist, mit Mensch oder Tier in Berührung gebracht zu werden, wie Badewasser, zur Konsumtion bestimmtes Wasser, für pharmazeutische oder biotechnologische Zubereitungen bestimmtes Wasser oder eine als Lebensmittel geeignete Flüssigkeit ist.

4. Verfahren nach einem der Ansprüche 1 bis 3, **dadurch gekennzeichnet, dass** das In-Berührung-Bringen der gegebenenfalls in der Flüssigkeit vorhandenen Mikroorganismen mit dem Substrat durch In-Berührung-Bringen der Flüssigkeit bzw. einer Probe dieser Flüssigkeit mit dem Substrat realisiert wird.

5. Verfahren nach einem der Ansprüche 1 bis 4, **dadurch gekennzeichnet, dass** das In-Berührung-Bringen der gegebenenfalls in der Flüssigkeit bzw. einer Probe der Flüssigkeit vorhandenen Mikroorganismen mit dem Substrat durch In-Berührung-Bringen eines Filtrats oder Zentrifugenrückstands der Flüssigkeit oder einer Probe dieser Flüssigkeit mit dem Substrat realisiert wird.

6. Verfahren nach einem der Ansprüche 1 bis 5, **dadurch gekennzeichnet, dass** die Flüssigkeit, die Flüssigkeitsprobe, oder das Konzentrat vor der Messung der Aktivität mindestens eines Enzyms einer aufschließenden Behandlung, insbesondere durch Beschallung, unterworfen wird.

7. Verfahren nach einem der Ansprüche 1 bis 6, **dadurch gekennzeichnet, dass** das/die Enzym/e, dessen/deren Aktivität gemessen wird/werden, in der Flüssigkeit, der Flüssigkeitsprobe oder dem Konzentrat ein Verhältnis von wahrer zu Anfangsaktivität in affiner Relation und mit einem Anstieg, der sich deutlich von Null unterscheidet und vorzugsweise kleiner als -0,2 ist, zu dem An-teil an überlebenden Mikroorganismen über mindestens einen Wertebereich des Anteils an überlebenden Mikroorganismen aufweist/aufweisen.

8. Verfahren nach einem der Ansprüche 1 bis 7, **dadurch gekennzeichnet, dass** das Enzym oder mindestens eines der Enzyme, dessen/deren Aktivität gemessen wird, eine Glucose-6-phosphat-Dehydrogenase, Malat-Dehydrogenase, Glycerinaldehyd-3-phosphat-Dehydrogenase, Katalase, Superoxid-Dismutase oder Glutathion-Reduktase ist.

9. Verfahren nach einem der Ansprüche 1 bis 8, **dadurch gekennzeichnet, dass** die Messungen der wahren und der Anfangsaktivität mittels eines Spektralphotometers, Spektralfluorometers oder Luminometers durchgeführt werden, die gegebenenfalls mehrere Analysekanäle besitzen.

10. Verfahren nach einem der Ansprüche 1 bis 9, **dadurch gekennzeichnet, dass** das Ausdrücken als Anteil an überlebenden Mikroorganismen mit Hilfe des Referenzsystems die Berechnung des gegebenenfalls in Prozent angegebenen Verhältnisses von wahrer zu Anfangsaktivität für das jeweilige Enzym umfasst.

11. Verfahren nach einem der Ansprüche 1 bis 10, **dadurch gekennzeichnet, dass** das Referenzsystem in Form eines Diagramms wie einer oder mehrerer Kurven vorliegt, die für das jeweilige Enzym das Verhältnis von wahrer zu Anfangsaktivität in Relation zu den Werten des Anteils an überlebenden Mikroorganismen bringen.

12. Verfahren nach einem der Ansprüche 9 bis 11, **dadurch gekennzeichnet, dass** die spektroskopischen Messungen für jede gemessene Enzymaktivität bei konstanter Temperatur, insbesondere 25 °C, und bei konstanter Wellenlänge, insbesondere einer Wellenlänge von 240 bis 550 nm, beispielsweise 340 nm, durchgeführt werden.

13. Verfahren nach einem der Ansprüche 9 bis 12, **dadurch gekennzeichnet, dass** die spektroskopischen Messungen über einen Zeitabschnitt von etwa 30 min kontinuierlich oder diskontinuierlich durchgeführt werden.

14. Verfahren nach einem der Ansprüche 1 bis 13, **dadurch gekennzeichnet, dass** die Anpassung der Dosen des/der chemischen bzw. physikalischen Mittel/s durch Zugabe des "Desinfektionsmitteldosisäquivalents" erfolgt, das der Differenz zwischen dem gewünschten Anteil an überlebenden Mikroorganismen und dem in der Flüssigkeit, der Probe oder dem Konzentrat gemessenen Anteil an Mikroorganismen entspricht.

15. Verfahren nach Anspruch 14, **dadurch gekennzeichnet, dass** das Desinfektionsmitteldosisäquivalent derart ist, dass es von einer Referenzkurve abgelesen wird, die den Anteil an überlebenden Mikroorganismen in Abhängigkeit von der Desinfektionsmitteldosis, der die Flüssigkeit ausgesetzt ist, darstellt.

16. Verfahren nach einem der Ansprüche 1 bis 15, **dadurch gekennzeichnet, dass** die Flüssigkeit Mikroorganismen enthält, welche aus der Gruppe ausgewählt sind, die von den Gattungen *Escherichia, Alcaligenes, Bacillus, Flavobacterium, Methylobacterium, Pseudomonas, Klebsiella, Enterobacterium, Agrobacterium, Streptococcus, Micrococcus* und *Salmonella* gebildet wird.

17. Verfahren nach einem der Ansprüche 1 bis 16, **dadurch gekennzeichnet, dass** es ein automatisiertes Verfahren zur Desinfektion einer Flüssigkeit ist.

18. Verfahren nach einem der Ansprüche 1 bis 17, **dadurch gekennzeichnet, dass** der Anteil an überlebenden Mikroorganismen als Schwächungswert (Konzentration der überlebenden Mikroorganismen, bezogen auf die Anfangskonzentration der überlebenden Mikroorganismen, wie sie in Stufe 1 gemessen wird, als Logarithmus der Schwächung ($-\log_{10}$(Schwächung))) oder Kennzahl D (= log-Schwächung) ausgedrückt wird.

19. Verfahren nach einem der Ansprüche 1 bis 18, **dadurch gekennzeichnet, dass** die überlebenden Mikroorganismen kultivierbare und/oder nicht kultivierbare, aber lebensfähige Mikroorganismen sind.

20. Verfahren nach einem der Ansprüche 1 bis 19, **dadurch gekennzeichnet, dass** die chemischen bzw. physikalischen Mittel, die zur Desinfektion angewendet werden, aus der Gruppe ausgewählt sind, die aus Chlor und seinen Verbindungen, UV-Strahlung, Ozon, $H_2O_2$, Filtermembranen, Temperatur, Ultraschall und ionisierender Strahlung besteht.

## Claims

1. Method for adjusting the disinfecting of a liquid, **characterised in that** it comprises:

   A. at a stage of said disinfecting hereinafter designated stage 2, measurement of the activity of at least one enzyme by putting microorganisms possibly present in said liquid into contact with a substrate chosen as being capable of showing the activity of this or these enzyme (s), this enzymatic activity being called hereinafter characteristic activity,

B. at a stage designated hereinafter stage 1, prior to said stage 2, measurement of the activity of the same enzyme(s) as in A, this activity being called hereinafter initial activity,

C. translating, for each enzyme, said characteristic activity and initial activity into contents of surviving microorganisms in said liquid at stage 2 of said disinfecting, this being via a reference system predetermined with the aid of a sample of said liquid taken at said stage 1 and then exposed to increasing doses of disinfectant(s), as well as

D. adjusting, as a function of said contents of surviving microorganisms, the type and/or the doses of chemical or physical agent(s) used for said disinfecting.

2. Method according to claim 1, **characterised in that** the said stage 1 corresponds to a stage before disinfecting said liquid and **in that** said stage 2 corresponds to any stage of said disinfecting.

3. Method according to claim 1 or 2, **characterised in that** said liquid is a liquid intended to be in contact with a person or an animal such as bathing water, water intended for consumption, water intended for pharmaceutical or biotechnology preparations or a nutritional liquid.

4. Method according to any of claims 1 to 3, **characterised in that** said putting in contact of the microorganisms possibly present in said liquid with said substrate is achieved by putting said liquid or a sample of said liquid in contact with said substrate.

5. Method according to any of claims 1 to 4, **characterised in that** said putting in contact of the microorganisms possibly present in said liquid, or sample of liquid, with said substrate is achieved by putting a filtrate or a residue from centrifugation of said liquid or sample of liquid, into contact with said substrate.

6. Method according to any of claims 1 to 5, **characterised in that** said liquid, sample of liquid or concentrate, undergoes, prior to said measurements of the activity of at least one enzyme, a lysis treatment, in particular by sonication.

7. Method according to any of claims 1 to 6, **characterised in that** the enzyme(s) of which the activity/activities is/are measured has/have, in said liquid, sample of liquid or concentrate, an affine ratio between characteristic activity and initial activity with an inclination significantly removed from zero, preferably lower than -0.2, with the content of surviving microorganisms on at least one value zone of said content of surviving microorganisms.

8. Method according to any of claims 1 to 7, **characterised in that** the enzyme, or at least one of the enzymes, of which the activity/activities is/are measured is a glucose-6-phosphate dehydrogenase, a malate dehydrogenase, a glyceraldehyde-3-phosphate dehydrogenase, a catalase, a superoxide dismutase, or a glutathione reductase.

9. Method according to any of claims 1 to 8, **characterised in that** said measurements of characteristic activity and initial activity are made with a spectrophotometer, a spectrofluorometer, or a luminometer optionally having a plurality of analysis ducts.

10. Method according to any of claims 1 to 9, **characterised in that** said translating into contents of surviving microorganisms with said reference system comprises, for each enzyme, calculation of the ratio between characteristic activity and initial activity, optionally expressed as a percentage.

11. Method according to any of claims 1 to 10, **characterised in that** said reference system is presented in graphic form such as one or a plurality of curve (s) showing, for each enzyme, said ratio between characteristic activity and initial activity corresponding to the content of surviving microorganism values.

12. Method according to any of claims 9 to 11, **characterised in that** said spectrometric measurements are obtained for each enzymatic activity measured, at constant temperature, in particular at 25 °C and at constant wavelength, in particular at a wavelength between 240 and 550 nm, for example at 340 nm.

13. Method according to any of claims 9 to 12, **characterised in that** said spectrometric measurements are obtained continuously or discretely over a time interval of about 30 minutes.

14. Method according to any of claims 1 to 13, **characterised in that** said adjustment of the doses of chemical or physical agent(s) is made by adding the equivalent "dose of disinfectant(s)" corresponding to the difference between the content of surviving microorganisms observed and the content of microorganisms measured in said liquid, liquid sample, or concentrate.

**15.** Method according to claim 14, **characterised in that** said equivalent dose of disinfectant(s) is as read from a reference curve showing the content of surviving microorganisms as a function of the dose of disinfectant(s) to which said liquid is exposed.

**16.** Method according to any of claims 1 to 15, **characterised in that** said liquid comprises microorganisms selected from among the group made up of the type *Escherichia, Alcaligenes, Bacillus, Flavobacterium, Methylobacterium, Pseudomonas, Klebsiella, Enterobacterium, Agrobacterium, Streptococcus, Micrococcus, Salmonella*.

**17.** Method according to any of claims 1 to 16, **characterised in that** it is automated on a method of disinfecting liquid.

**18.** Method according to any of claims 1 to 17, **characterised in that** said content of surviving microorganisms is expressed as a reduction value (concentration of surviving microorganisms as a function of the initial concentration of surviving microorganisms as measured at said stage 1 from reduction log ($-\log_{10}$ (reduction)) or from index D (= reduction log).

**19.** Method according to any of claims 1 to 18, **characterised in that** said surviving microorganisms are cultivatable microorganisms and/or microorganisms which are non-cultivatable but viable.

**20.** Method according to any of claims 1 to 19, **characterised in that** said chemical or physical agents used for disinfecting are selected from the group made up by chlorine and its derivatives, UVs, ozone, $H_2O_2$, filter membranes, temperature, ultrasound, ionising radiation.

Figure 1

Figure 2

Figure 3